# FASCICULE DE BREVET EUROPEEN

(11) **EP 2 271 606 B1**
(45) Date de publication et mention de la délivrance du brevet: **05.10.2016**
(21) Numéro de dépôt: 09724425.5
(22) Date de dépôt: 27.03.2009
(51) Int. Cl.: C07C 21/18, C07C 17/25, B01J 37/26, B01J 23/86, B01J 21/04, B01J 35/10, B01J 37/02

(54) **PROCEDE POUR LA PREPARATION DU 1, 2, 3, 3, 3-PENTAFLUOROPROPENE-1**
VERFAHREN ZUR HERSTELLUNG VON 1,2,3,3,3-PENTAFLUORPROP-1-EN
METHOD FOR PREPARING 1, 2, 3, 3, 3-PENTAFLUOROPROPENE-1

(30) Priorité: 28.03.2008 FR 0801726
(43) Date de publication de la demande: 12.01.2011
(73) Titulaire: ARKEMA FRANCE, 92700 Colombes (FR)
(72) Inventeur: DEVIC, Michel, F-69110 Sainte Foy Les Lyon (FR); WENDLINGER, Laurent, F-69510 Soucieu en Jarrest (FR)
(74) Mandataire: Hirsch & Associés
(86) Numéro de dépôt international: PCT/IB2009/005089
(87) Numéro de publication internationale: WO 2009/118628

(56) Documents cités:
- EP-A- 0 644 173
- EP-B- 0 974 571
- WO-A-94/27940
- WO-A-2006/106353
- WO-A-2007/117391
- WO-A-2008/030440
- WO-A-2008/030444

## Description

### DOMAINE DE L'INVENTION

L'invention a pour objet un procédé de préparation de composés fluorés, notamment le composé fluoré 1225ye.

### ARRIERE-PLAN TECHNOLOGIQUE

Les hydrofluorocarbones (HFC) et en particulier les hydrofluoropropènes sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

WO 2007/056194 décrit la préparation de HFO par conversion d'un composé de formule (I) CF₃CHXCH₂X en un composé de formule (II) CF₃CZCHZ, formules dans lesquelles X est indépendamment Cl ou F, et Z est indépendamment H ou F. La réaction a lieu en phase gaz, sur un catalyseur. Les catalyseurs décrits dans cette demande sont des catalyseurs par exemple FeCl₃, oxyfluorure de chrome, Ni (incluant les treillis de Ni mesh), NiCl₂, CrF₃, et leurs mélanges. D'autres catalyseurs décrits sont les catalyseurs supportés sur carbone, les catalyseurs à base d'antimoine, les catalyseurs à base d'aluminium (tel que AlF₃ et Al₂O₃), de palladium, de platine, de rhodium et de ruthénium. Un mode de réalisation préféré est la préparation de 1234yf par déhydrohalogénation de 245eb, notamment sur catalyseur à base de nickel, de carbone ou une combinaison de ceux-ci. La température indiquée comme étant appropriée est comprise entre 450°C et 600°C.

Le document WO 2007/056128 décrit la réaction de déhydrohalogénation de composés de formule (I) CF₃CFₙCHₘXₐ₋ₘ en un composé de formule (II) CF₃CZ=CHZ. Cette réaction est notamment conduite en présence d'un agent réducteur, l'hydrogène.

De même, on connaît les réactions de déhydrofluoration de 1,1,1,2,3,3-hexafluoropropane (HFC 236ea) en 1,2,3,3,3-pentafluoropropène-1 (1225ye).

Le document US-P-5396000 décrit la préparation de 1,1,1,2,3-pentafluoropropane par déhydrohalogénation catalytique de 1,1,1,2,3,3-hexafluoropropane (236ea) en 1,2,3,3,3-pentafluoropropène-1 (1225ye), suivi d'une hydrogénation pour produire le composé recherché. La déshydrohalogénation du 236ea en 1225ye est effectuée en phase gazeuse, sur un catalyseur à base d'alumine fluorée. La sélectivité en 1225ye n'est pas indiquée.

La demande internationale WO 94/27940 décrit la préparation de 1225ye à partir de 236ea en phase vapeur en présence d'un catalyseur choisi dans le groupe des fluorures d'aluminium ou métal supporté sur fluorure d'aluminium, le choix du métal pouvant être le chrome. Le 1225ye est utilisé pour la préparation de 245eb. Cependant, dans la réaction de déhydrofluoration, aucun autre catalyseur n'est mentionné dans cette référence.

La demande internationale WO 2007/117391 décrit la préparation de 1225ye et 1234yf. Un mélange de 1,1,1,2,3,3-hexafluoropropane (236ea) et 1,1,1,2,3-pentafluoropropane (245eb) est mis en présence d'un catalyseur (tel que métaux sur alumine fluorée, oxydes, oxydes de chrome trifluorure de Cr^{III}, charbon actif). Aucune information n'est donnée vis-à-vis du résultat attendu selon les catalyseurs choisis (conversion, sélectivité, ou rendement). La déhydrofluoration est mise en oeuvre sous azote.

La demande internationale WO 2008/030444 concerne un autre produit de départ (le 236cb et non le 236ea). Le 236cb ne présente pas de problème de sélectivité. De l'hydrogène non éliminé, venant de l'étape précédente, peut se trouver introduit mais l'hydrogène n'intervient pas dans la réaction.

La demande internationale WO 2008/030440 concerne la préparation de CH₂=CF-CF₃ par addition d'hydrogène à CHF=CF-CF₃ (1225yf) en présence d'un catalyseur d'hydrogénation, pour préparer CH₂F-CHF-CF₃ suivi de déhydrohalogénation du produit obtenu, en phase vapeur, en présence d'un catalyseur (tel que fluorures d'aluminium, métals sur fluorures d'aluminium, oxydes, les fluorures ou oxyfluorures de magnésium ou zinc ou oxydes de chrome ou composés métalliques supportés sur carbone), pour produire CH₂=CF-CF₃. Le 1225ye est le produit de départ, il n'est pas décrit de déhydrofluoration à partir du 236ea.

La demande internationale EP 974,571 décrit un procédé de déhydrofluoration de 1,1,1,3,3-pentafluoropropane (245fa) pour la préparation de cis/trans 1,3,3,3 tetrafluoro-1-propene (1234ze) en présence d'un catalyseur à base de chrome qui peut être un catalyseur mixte à base de chrome et de nickel sur un support d'aluminium tel que le fluorure d'aluminium. Il s'agit de déhydrofluoration d'autres produits (245fa), aucun problème de sélectivité n'intervient.

Le document US-P-5679875 décrit la préparation de 1,1,1,2,3-pentafluoropropane par déhydrohalogénation catalytique de 1,1,1,2,3,3-hexafluoropropane (236ea) en 1,2,3,3,3-pentafluoropropène-1 (1225ye), suivi d'une hydrogénation pour produire le composé recherché. Les réactions sont effectuées en phase gaz. Le catalyseur de déhydrohalogénation est l'oxyde ou oxyfluorure de chrome III, ou du carbone. De l'oxygène peut être ajouté au courant de 1,1,1,2,3,3-hexafluoropropane pour maintenir l'activité catalytique, ainsi que la sélectivité, au cours du temps. Les sélectivités sont élevées, mais la conversion n'est pas toujours très élevée et n'est pas nécessairement constante dans le temps.

Il existe donc un besoin d'un procédé de préparation de fluorooléfines, et ce avec une sélectivité élevée pour une conversion élevée, et par là un rendement élevé. Ce procédé devrait aussi avantageusement permettre une stabilité dans le temps, notamment en terme de sélectivité. Ce besoin est notamment vrai pour les réactions de préparation de 1225ye à partir de 236ea.

### RESUME DE L'INVENTION

- L'invention fournit donc un procédé de déhydrofluoration de 1,1,1,2,3,3-hexafluoropropane en 1,2,3,3,3-pentafluoropropène-1, en présence d'hydrogène et dans lequel le catalyseur de déhydrofluoration est un catalyseur mixte à base de chrome et de nickel sur un support à base d'aluminium.

Selon des modes de réalisation:
- la réaction est mise en oeuvre en phase gazeuse avec un ratio molaire H₂/1,1,1,2,3,3-hexafluoropropane compris entre 0,3 et 30.
- la réaction est mise en oeuvre en phase gazeuse avec un ratio molaire H₂/1,1,1,2,3,3-hexafluoropropane compris entre 0,5 et 20, avantageusement entre 1 et 10.
- le procédé est mis en oeuvre en présence d'un catalyseur de déhydrofluoration.
- le procédé est mis en oeuvre à une température comprise entre 150°C et 500°C, de préférence entre 300 et 400°C.
- le procédé est mis en oeuvre avec un temps de contact compris entre 0,1 et 100 secondes, de préférence entre 1 et 50 secondes et avantageusement entre 5 et 40 secondes.

### DESCRIPTION DETAILLEE DE MODES DE REALISATION

L'invention est basée sur la découverte que la réaction de déhydrofluoration, lorsqu'elle est mise en oeuvre en présence d'hydrogène, offre une sélectivité élevée en le produit recherché, et ce de façon stable dans le temps, tant au niveau conversion que sélectivité.

Le produit de départ est le CF₃-CHF-CHF₂ (F236ea). Dans ce cas, l'élimination de HF peut conduire à deux produits, le premier qui est le produit recherché CF₃-CF=CHF (1225ye) tandis que le produit non recherché correspond à la formule CF₃-CH=CF₂ (1225zc), selon le fluor qui est éliminé. Il y a donc un problème de sélectivité lors de l'élimination de HF de la molécule de départ. Un tel problème de sélectivité ne se pose pas si le produit de départ ne présente pas de fluor sur le carbone terminal destiné à porter la double liaison.

La sélectivité vers le produit recherché est très élevée, supérieure à 90%, de préférence supérieure à 95% et même avantageusement supérieure ou égale à 98%.

La conversion est aussi très élevée. Notamment la conversion reste élevée dans le temps.

La productivité obtenue avec le procédé selon l'invention est élevée, et avantageusement reste stable dans le temps.

En général, de l'hydrogène est injecté avec la charge de départ, par exemple en continu ou de façon alternée. Le ratio molaire H₂/charge départ peut varier dans de grandes mesures, notamment entre 0,3 et 30, notamment entre 0,5 et 20, avantageusement entre 1 et 10.

La mise en oeuvre de la réaction de déhydrofluoration en présence d'hydrogène permet donc une sélectivité élevée, et avantageusement une conversion élevée, stables dans le temps. La présence d'hydrogène permet aussi de réduire la production de lourds au cours de la réaction.

Par ailleurs, dans le cas de la production du composé 1225ye, le ratio entre les isomères Z et E, Z/E, est en général compris entre 5 et 10, de préférence entre 6 et 8. La réaction de déhydrofluoration est mise en oeuvre avec un catalyseur de déhydrofluoration particulier, dont l'utilisation dans les réactions de déhydrofluoration sélective n'est pas décrite dans l'état de la technique.

Ce catalyseur est mixte, contenant à la fois du chrome et du nickel. Le ratio molaire Cr:Ni, par rapport à l'élément métallique, est généralement compris entre 0,5 et 5, par exemple entre 0,7 et 2, notamment voisin de 1. Le catalyseur peut contenir en poids de 0,5 à 20% de chrome et de 0,5 à 20% de nickel et, de préférence, entre 2 et 10% de chacun des métaux.

Le métal peut être présent sous forme métallique ou sous forme de dérivés, notamment oxyde, halogénure ou oxyhalogénure, ces dérivés, notamment halogénure et oxyhalogénure, étant obtenus par activation du métal catalytique. Bien que l'activation du métal ne soit pas nécessaire, elle est préférée.

Le support est à base d'aluminium. On peut citer plusieurs supports possibles comme l'alumine, l'alumine activée ou les dérivés d'aluminium. Ces dérivés d'aluminium sont notamment des halogénures ou oxyhalogénures d'aluminium, décrits par exemple dans US-P-4902838, ou obtenus par le procédé d'activation décrit ci-dessous.

Le catalyseur peut comprendre le chrome et du nickel sous une forme non-activée ou sous forme activée, sur un support qui a subi aussi l'activation du métal ou non.

Le catalyseur peut être préparé à partir d'alumine (en général une alumine dite activée; cette alumine activée est une alumine à porosité élevée, et est distincte de l'alumine ayant subi le traitement d'activation du métal). Dans une première étape l'alumine est transformée en fluorure d'aluminium ou en mélange de fluorure d'aluminium et d'alumine, par fluoration à l'aide d'air et d'acide fluorhydrique, le taux de transformation de l'alumine en fluorure d'aluminium dépendant essentiellement de la température à laquelle est effectuée la fluoration de l'alumine (en général entre 200°C et 450°C, de préférence entre 250°C et 400°C). Le support est ensuite imprégné à l'aide de solutions aqueuses de sels de chrome et de nickel ou à l'aide de solutions aqueuses d'acide chromique, de sel de nickel et de méthanol (servant de réducteur au chrome). Comme sels de chrome et de nickel, on peut employer des chlorures, ou d'autres sels tels que, par exemple, les oxalates, formiates, acétates, nitrates et sulfates ou le bichromate de nickel, pour autant que ces sels soient solubles dans la quantité d'eau susceptible d'être absorbée par le support. Le catalyseur peut aussi être préparé par imprégnation directe de l'alumine (qui en général est activée) à l'aide des solutions des composés de chrome et de nickel ci-dessus mentionnées. Dans ce cas, la transformation d'au moins une partie (par exemple 70% ou plus) de l'alumine en fluorure d'aluminium ou oxyfluorure d'aluminium s'effectue lors de l'étape d'activation du métal du catalyseur.

Les alumines activées susceptibles d'être utilisées pour la préparation du catalyseur sont des produits bien connus, disponibles dans le commerce. Elles sont généralement préparées par calcination d'hydrates d'alumine (hydroxydes d'aluminium) à une température comprise entre 300°C et 800°C. Les alumines (activées ou non) peuvent contenir des teneurs importantes (jusqu'à 1000 ppm) de sodium sans que cela nuise aux performances catalytiques.

De préférence, mais sans que cela soit nécessaire, le catalyseur est conditionnée ou activé, c'est-à-dire transformé en constituants actifs et stables (aux conditions réactionnelles) par une opération préalable dite d'activation. Ce traitement peut être réalisé soit "in situ" (dans le réacteur de déhydrofluoration) ou bien dans un appareillage adéquat conçu pour résister aux conditions d'activation.

Cette étape d'activation comprend en général les étapes suivantes:
- Une étape de séchage. Cette étape de séchage est effectuée à haute température (250°C à 450°C, de préférence 300°C à 350°C) en général sous courant d'azote ou d'air. Cette étape peut être éventuellement précédée dans un premier temps d'une première étape de séchage à basse température (100°C à 150°C, de préférence 110°C à 120°C) en présence d'air ou d'azote. La durée de l'étape de séchage peut être comprise entre 1 et 50 heures.
- Une étape de fluoration. Cette étape de fluoration est mise en oeuvre à basse température (180°C à 350°C) au moyen d'un mélange d'acide fluorhydrique et d'azote, en contrôlant la teneur en HF de façon que la température ne dépasse pas 350°C. La durée de l'étape de fluoration peut être comprise entre 1 et 50 heures.
- Eventuellement une étape de finition sous courant d'acide fluorhydrique pur ou dilué par de l'azote à une température pouvant aller jusqu'à 450°C. La durée de l'étape de finition peut être comprise entre 1 et 15 heures.

Pendant cette opération, les précurseurs catalytiques (par exemple halogénures de nickel et de chrome, chromate ou bichromate de nickel, oxyde de chrome) sont transformés en fluorures et/ou oxyfluorures correspondants, ce qui entraîne un dégagement d'eau et/ou d'acide chlorhydrique. L'analyse chimique des éléments (chrome, nickel, fluor, aluminium, oxygène), après cette activation, permet de vérifier la composition minérale du catalyseur.

Un tel catalyseur est décrit dans EP-A-486333, en particulier page 3, lignes 11-48, exemples 1A, 2A et 4A, passages auxquels il est renvoyé.

La réaction de déhydrofluoration est mise en oeuvre en général en phase gazeuse.

Le catalyseur peut être présent sous toute forme appropriée, par exemple sous forme de lit fixe ou fluidisé, de préférence en lit fixe. La direction de flux peut être de haut en bas ou de bas en haut.

La température peut être comprise entre 150°C et 600°C, de préférence entre 300 et 500°C et avantageusement entre 300 et 450°C, notamment entre 300 et 400°C.

La pression peut être atmosphérique, ou inférieure ou supérieure à cette pression atmosphérique.

Le temps de contact (rapport entre le volume de catalyseur et le flux total de la charge) est en général compris entre 0,1 et 100 secondes, de préférence entre 1 et 50 secondes et avantageusement entre 5 et 40 secondes.

Un gaz diluant (azote, hélium ou argon) peut être utilisé dans la réaction.

La réaction est mise en oeuvre dans un réacteur dédié aux réactions impliquant des halogènes. De tels réacteurs sont connus de l'homme du métier, et peuvent comprendre des revêtements intérieurs à base par exemple de Hastelloy®, Inconel®, Monel® ou de fluoropolymères. Le réacteur peut aussi comprendre des moyens d'échange de chaleur, si besoin.

L'alimentation en réactifs peut se faire en général en continu, ou peut être étagée le cas échéant.

Le produit final de la réaction est séparé de façon classique, et les réactifs n'ayant pas réagi sont avantageusement recyclés dans le procédé. On rappellera que:
- le taux de conversion est le % du produit de départ ayant réagi (nombre mole de produit départ ayant réagi/nombre mole produit départ introduit);
- la sélectivité en produit recherché est le ratio nombre de mole de produit recherché formé/nombre mole produit départ ayant réagi;
- le rendement en produit recherché est le ratio nombre de mole de produit recherché formé/nombre mole produit départ introduit, le rendement en produit recherché pouvant encore être défini comme le produit de la conversion et de la sélectivité.
- le temps de contact est l'inverse de la vitesse spatiale VVH (ou GHSV en langue anglaise)
- la vitesse spatiale est le rapport entre le débit volumique total sur le volume du lit catalytique, dans les conditions normales de température et de pression.
- La productivité est exprimée en masse de produit recherché obtenu par unité de temps et par unité de catalyseur (masse ou volume); cette productivité est liée au temps de contact.

### EXEMPLES

Les exemples suivants illustrent l'invention sans la limiter. Exemple 1. Préparation du catalyseur.

Le catalyseur utilisé est un catalyseur Ni-Cr/AlF₃ préparé comme suit.

Dans un évaporateur rotatif, on place 343 g d'un support obtenu dans une étape précédente par fluoration d'alumine GRACE HSA en lit fixe vers 280°C à l'aide d'air et d'acide fluorhydrique (concentration volumique de 5 à 10% de cet acide dans l'air). L'alumine GRACE HSA de départ présente les caractéristiques physicochimiques suivantes:

| | |
|---|---|
| - Forme | : billes de 0,5-2 mm de diamètre |
| - surface BET | : 220 m²/g |
| - volume poreux | : 1, 3 cm³/g |

On prépare par ailleurs deux solutions aqueuses séparées:
(a) solution chromique additionnée de chlorure de nickel contenant:

| | |
|---|---|
| - anhydride chromique | 55 g |
| - chlorure de nickel hexahydraté | 130 g |
| - eau | 63 g |

(b) Solution méthanolique contenant:

| | |
|---|---|
| - méthanol | 81 g |
| - eau | 8 g |

Ces deux solutions sont introduites simultanément à une température de 40°C sous pression atmosphérique et en 2 heures environ, sur le support en agitation. Après une étape de maturation sous azote, le catalyseur est séché sous azote, puis sous vide à 65°C puis vers 90°C pendant 6 heures.

On charge 500 g de solide imprégné dans un réacteur tubulaire en inconel. Le catalyseur est tout d'abord séché sous balayage d'azote à basse température puis jusqu'à 320°C, à pression atmosphérique. Il est ensuite fluoré en présence d'un mélange acide fluorhydrique / azote (concentration volumique de 5 à 10% de cet acide dans l'azote) à 320°C puis jusqu'à 390°C. L'alimentation d'HF est alors coupée. Le balayage à l'azote est maintenu pendant 15 minutes à 390°C puis le catalyseur est refroidi jusqu'à 60°C sous balayage d'azote.

Les caractéristiques du catalyseur après activation sont les suivantes:

| | |
|---|---|
| - surface BET | : 40 m²/g |
| - volume poreux | : 0, 4 cm³/g |
| - composition chimique pondérale: | |
| ▪ Al | : 25 % |
| ▪ F | : 58 % |
| ▪ Cr | : 5,3 % |
| ▪ Ni | : 6,4 % |

### Exemple 2. Déhydrofluoration de 236ea en 1225ye.

On utilise un réacteur d'un volume de 50 cm³ et de 2,1 cm de diamètre, contenant 20 g de catalyseur sous forme d'un lit fixe d'une hauteur de 6,5 cm. La pression est de 1 bar.

Les réactifs sont mélangés avec l'hydrogène et injectés dans le réacteur préalablement chauffé à la température de réaction.

On obtient alors les résultats suivants (RM signifie ratio molaire).

| Temp. | Temps contact | RM H₂/236ea | Conversion | Sélectivité 1225ye | Productivité 1225ye Kg/h/m³ cata |
|---|---|---|---|---|---|
| 375°C | 10,4 sec | 2,14 | 50,1% | 97,6% | 319 |

Au bout de 100 heures de fonctionnement, les performances restent constantes.

## Revendications

1. Procédé de déhydrofluoration de 1,1,1,2,3,3-hexafluoropropane en 1,2,3,3,3-pentafluoropropène-1, mis en oeuvre en présence d'un catalyseur de déhydrofluoration qui est un catalyseur mixte à base de chrome et de nickel sur un support à base d'aluminium, en présence d'hydrogène.

2. Procédé selon la revendication 1, dans lequel la réaction est mise en oeuvre en phase gazeuse avec un ratio molaire H₂/1,1,1,2,3,3-hexafluoropropane compris entre 0,3 et 30.

3. Procédé selon la revendication 1 ou 2, dans lequel la réaction est mise en oeuvre en phase gazeuse avec un ratio molaire H₂/1,1,1,2,3,3-hexafluoropropane compris entre 0,5 et 20, avantageusement entre 1 et 10.

4. Procédé selon l'une des revendications 1 à 3, mis en oeuvre à une température comprise entre 150°C et 500°C, de préférence entre 300 et 400°C.

5. Procédé selon l'une des revendications 1 à 4, mis en oeuvre avec un temps de contact compris entre 0,1 et 100 secondes, de préférence entre 1 et 50 secondes et avantageusement entre 5 et 40 secondes.

## Patentansprüche

1. Verfahren zur Dehydrofluorierung von 1,1,1,2,3,3-Hexafluorpropan in 1,2,3,3,3-Pentafluorpropen-1, welches in Anwesenheit eines Dehydrofluorierungskatalysators, der ein gemischter Katalysator auf der Basis von Chrom und Nickel auf einem Träger auf der Basis von Aluminium ist, in Anwesenheit von Wasserstoff durchgeführt wird.

2. Verfahren nach Anspruch 1, wobei die Reaktion in der Gasphase mit einem Molverhältnis H**₂**/1,1,1,2,3,3-Hexafluorpropan zwischen 0,3 und 30 durchgeführt wird.

3. Verfahren nach Anspruch 1 oder 2, wobei die Reaktion in der Gasphase mit einem Molverhältnis H₂/1,1,1,2,3,3-Hexafluorpropan zwischen 0,5 und 20, vorteilhaft zwischen 1 und 10, durchgeführt wird.

4. Verfahren nach einem der Ansprüche 1 bis 3, welches bei einer Temperatur zwischen 150 °C und 500 °C, vorzugsweise zwischen 300 und 400°C, durchgeführt wird.

5. Verfahren nach einem der Ansprüche 1 bis 4, welches mit einer Kontaktzeit zwischen 0,1 und 100 Sekunden, vorzugsweise zwischen 1 und 50 Sekunden, und vorteilhaft zwischen 5 und 40 Sekunden, durchgeführt wird.

## Claims

1. A process for dehydrofluorination of 1,1,1,2,3,3-hexafluoropropane into 1,2,3,3,3-pentafluoropropene-1, carried out in the presence of a dehydrofluorination catalyst which is a mixed catalyst based on chromium and nickel on an aluminum-based support, in the presence of hydrogen.

2. Process according to claim 1, wherein the reaction is carried out in the gaseous phase with an H₂/1,1,1,2,3,3-hexafluoropropane molar ratio of between 0.3 and 30.

3. Process according to claim 1 or 2, wherein the reaction is carried out in the gaseous phase with an H₂/1,1,1,2,3,3-hexafluoropropane molar ratio of between 0.5 and 20, advantageously between 1 and 10.

4. Process according to one of claims 1 to 3, carried out at a temperature of between 150°C and 500°C, preferably between 300 and 400°C.

5. Process according to one of claims 1 to 4, carried out with a contact time of between 0.1 and 100 seconds, preferably between 1 and 50 seconds and advantageously between 5 and 40 seconds.
